(19) Europäisches Patentamt
European Patent Office
Office européen des brevets

(11) Publication number: 0 171 762 B1

# (12) EUROPEAN PATENT SPECIFICATION

(45) Date of publication of patent specification: 18.07.90

(51) Int. Cl.$^5$: C 07 C 201/12, C 07 C 205/57

(21) Application number: 85110001.6

(22) Date of filing: 08.08.85

(54) **Process for preparing carboxylate esters.**

(30) Priority: 13.08.84 US 639797

(43) Date of publication of application: 19.02.86 Bulletin 86/08

(45) Publication of the grant of the patent: 18.07.90 Bulletin 90/29

(84) Designated Contracting States: DE FR GB NL SE

(56) References cited:
GB-A- 916 772
GB-A- 966 266
US-A-3 879 445
US-A-3 891 693
US-A-3 992 432
US-A-4 476 318

Charles M. STARKS and Charles LIOTTA "Phase Transfer Catalysis", Principles and Techniques, Academic Press, 1978; pp. 140-155

(73) Proprietor: AIR PRODUCTS AND CHEMICALS, INC.
7201 Hamilton Boulevard
Allentown, PA 18195-1501 (US)

(72) Inventor: Seekings, Sara M.
39 Walcott Valley Drive
Hopkinton, MA 02360 (US)
Inventor: Whitten, Charles E.
12 Lisa Avenue
Plymouth, MA 02360 (US)

(74) Representative: Reitzner, Bruno, Dr. et al
Patentanwälte Dipl.-Ing. R. Splanemann Dr. B. Reitzner, Dipl.-Ing. K. Baronetzky Tal 13
D-8000 München 2 (DE)

(56) References cited:
TETRAHEDRON LETTERS, no. 27, 1975; K.HOLMBERG et al.: "Ethylene diesters of carboxylic acids from dichloromethane", pp. 2303-2306

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 171 762 B1

(56) References cited:
E.V. and S.S. DEHMLOW: "Phase transfer catalysis", Verlag Chemie, 1980; pp. 52, 69-77

I & EC PRODUCT RESEARCH & DEVELOPMENT, vol. 7, no. 2, 1968; H.E.HENNIS et al.: "Esters from the reactions of alkylhalides and salts of carboxylicacids", pp. 96-101

SYNTHETIC COMMUNICATIONS, no. 2(4), 1972; J.H.WAGENKNECHT et al.: "A rapid mild esterification method", pp. 215-219

OFFICIAL JOURNAL CHEM. IND., London, 1962; R.L.MERKER et al.: "The reaction of alkylhalides with carboxylic acids and phenols in the presence of tertiary amines", pp. 5180-5182

CHEMICAL ABSTRACTS, vol. 53, 1959; p. 9025

SYNTHESIS, Dec 1974; T.TORU et al.: "New synthesis of cis-3,5-diacetoxycyclopentene", pp. 867

## Description

### Background of the Invention

This invention relates to a method for the production of carboxylate esters. More particularly, it relates to a method for the production of carboxylate diesters by the reaction of a carboxylate metal salt and a dihaloalkane.

The production of esters by the reaction of a salt of a carboxylic acid and an alkyl halide is a well known textbook method. The reaction of simple alkyl halides with alkali metal salts of carboxylic acids in suitable solvents to produce esters has, however, been described as being of little preparative value, owing to poor yields and conversions along with competing side reactions of dehydrohalogenations (Mills, R. H., Farrar, M. W., Weinkauff, O. J., Chem. Ind., London, 1962, p. 2144). The manufacture of carboxylate esters by resort to such methodology has been hampered in part by the absence of suitable solvent for the alkyl halide and alkali metal carboxylate reactants which exhibit considerable incompatability. Moreover, utilization of a suitable mutual solvent medium for the reactants requires recovery of the solvent material for efficient production of the desired ester product.

In U.S. Patent, 3,932,360 (issued January 13, 1976 to L. D. Cerankowski et al.) there is described a class of amino-containing diester curing agents useful in the production of polyurethane products. These diesters are prepared by reacting p-nitrobenzoyl chloride with a diol; and reducing the nitro groups of the resulting diester to amino groups. The esterification reaction is conducted in a mutual solvent which, in an efficient production method, requires recovery and recycling.

In Japanese Patent No. 57—81445 (Application No. 55—157868, published May 21, 1982) there is described a method for producing a bis (aminobenzoate) diester by reaction of a dihalide, an amino benzoate and a base. The reaction is conducted in a solvent which promotes agitation and reaction. In Japanese Patent No. 58—59949 (Application No. 56—159596, published April 9, 1983) there is disclosed a process for producing 1,3-propanediol bis(p-aminobenzoate) by reacting p-aminobenzoate alkali metal salt with dihalogenated propane, in an aprotic dipolar solvent. These methods require separation of the desired product and recovery of the solvent medium.

It will be appreciated that a process for the efficient production of carboxylate diester by the reaction of a carboxylate metal salt and a dihaloalkane, and without the requirement for an extraneous organic solvent or the recovery thereof, will offer considerable advantage.

### Summary of the Invention

It has been found that dicarboxylate esters can be conveniently formed by the reaction of a dihaloalkane and a carboxylate metal salt by resort to a phase-transfer catalyzed reaction involving aqueous and organic phases wherein the dihaloalkane functions as both a reactant and a solvent medium and that the desired product can be formed without the need for an extraneous organic solvent or the recovery thereof.

According to the present invention, there is provided a process for preparing a diester having the formula (I)

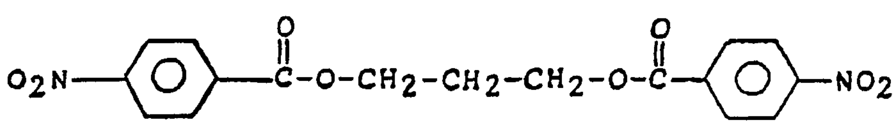

said process comprising reacting, in an aqueous-and-organic two-phase reaction medium, a reactant salt of the formula (II)

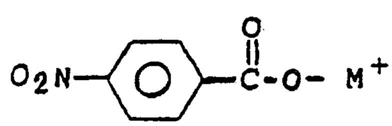

wherein $M^+$ is an alkali metal, and substantially an equivalent amount of a dihaloalkane reactant of the formula (III)

$$X^1—CH_2—CH_2—CH_2—X^2$$

wherein each of $X^1$ and $X^2$ is halo; said aqueous-and-organic two-phase reaction medium being formed without an extraneous organic solvent by contacting water, said reagent salt and said dihaloalkane reagent, and being maintained during reaction by the formation of said diester reaction product; said reaction in said two-phase medium being conducted in the presence of a phase-transfer catalyst.

Various objects, details, operations, uses, advantages and modifications of the invention will be apparent from the following detailed description of the present invention.

## Detailed Description of the Invention

As aforesaid, the process of the present invention provides a carboxylate diester of the formula (I) shown above.

The desired product is simply and efficiently produced in good yield and purity.

In the production of the diester according to the process of the invention, an aqueous-and-organic two-phase reaction medium is prepared. This is readily acocmplished, for example, by contacting an aqueous solution of the reactant salt of the formula II shown above, wherein $M^+$ represents an alkali metal (e.g. sodium, potassium), with the dihaloalkane reactant of the formula III as shown above, wherein each of $X^1$ and $X^2$ may, for example, be chloro or bromo. The mutual incompatibility of the aqueous solution of the carboxylate and the organic dihaloalkane reactant is such that a two-phase system is produced. Thus, the dihaloalkane functions as both a reactant for the production of the desired diester of formula (I) and as a reaction solvent for the provision of the requisite two-phase medium of a phase-transfer catalyzed reaction.

Preferably, from the standpoint of desired rate of reaction, at least one of the X moieties of the dihaloalkane of formula (III) will be bromo. A preferred dihaloalkane is 1-bromo-3-chloropropane, although other dihaloalkanes can be employed. The preferential reactivity of a bromo moiety, compared with a chloro moiety, makes the utilization of a bromo-chloroalkane reactant advantageous from the standpoint of desired rate of reaction. For example, the reaction of 1-bromo-3-chloropropane with p-nitrobenzoate (sodium salt) can be accomplished via a phase-transfer catalyzed reaction over a period of about four hours or less at a temperature of about 100°C. This compares, for example, with a reaction time of about 12 to 16 hours using the 1,3-dichloropropane reactant.

In the conduct of the phase-transfer catalyzed process of the invention, an intermediate monoester product of the following formula (IV)

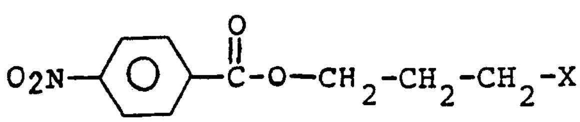

(IV)

is believed to be produced. This product and the desired diester product of formula (I), formed by the reaction of the monoester and additional carboxylate, serve during the reaction to maintain the organic phase of the requisite two-phase reaction system and permit the phase-transfer catalyzed process to be completed, notwithstanding the depletion of the dihaloalkane reactant which is employed initially in the formation of the two-phase reaction system.

The production of diesters according to the process of the invention can be illustrated by the following equation which is a representation of a preferred embodiment.

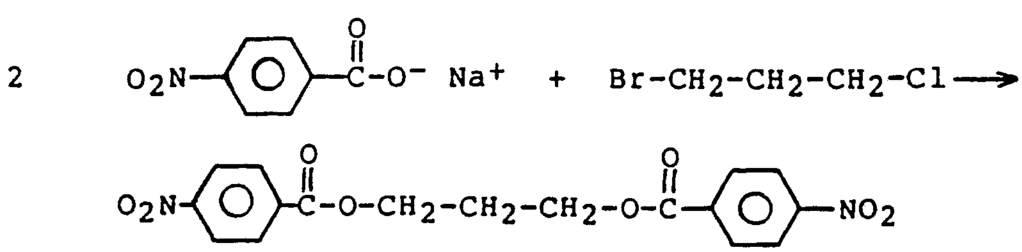

While applicant does not wish to be bound by any particular mechanism by which the process of the invention provides the desired esters hereof, the following equations are presented as illustrating reactions which are believed to occur in the production thereof. In the following equations, Ar represents the p-nitrophenyl radical:

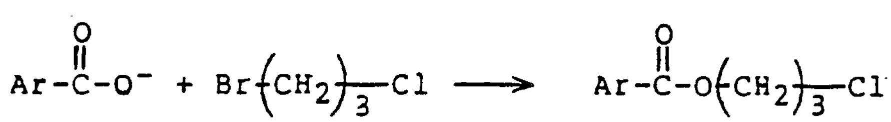

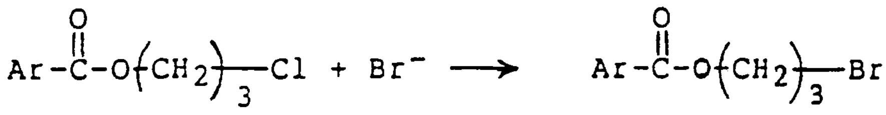

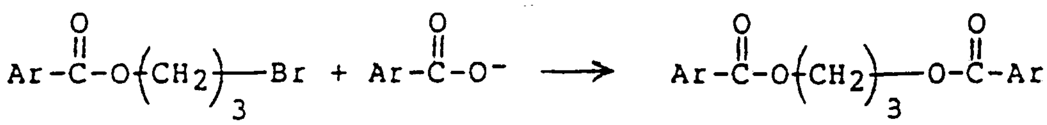

The reactions as described above are effected by the employment of a phase-transfer catalyst which serves to transfer water-soluble reactant across the interface and into the organic reactant phase. This can be illustrated by the following representation wherein $Q^+Br^-$ represents a phase-transfer catalyst and $Q^+$ is a quaternary ion such as quaternary ammonium or phosphonium:

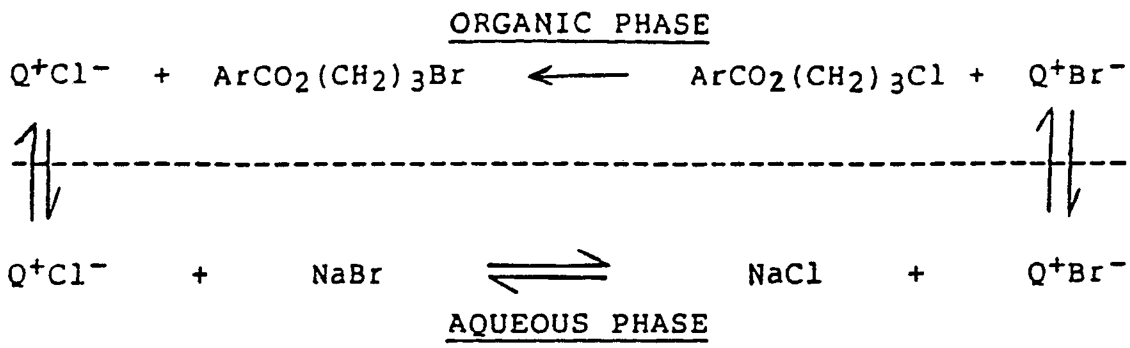


In the preparation of the carboxylate diesters according to the process of the invention, any of a variety of phase-transfer catalysts can be used. Suitable phase-transfer catalysts are those agents which are effective to transfer the water-soluble reactant across the interface into the organic phase where reaction occurs. Thus, agents which form an ion-pair with the water-soluble carboxylate nucleophile so that the ion pair is transferred into the organic phase for reaction with the organic dihalide (or the formula (IV) monohaloalkyl monoester) can be employed. Quaternary ammonium and phosphonium halide salts can be used for this purpose with good results. Examples include tetrabutylammonium, methyltrioctylammonium, and tetrabutylphosphonium salts and tributylmethylammonium chloride, tetrabutylammonium bromide, trioctylmethylammonium chloride, hexadecyltrimethylammonium chloride, and the corresponding phosphonium salts. The tetraalkylammonium halide (e.g., chloride or bromide) salts are particularly useful. Preferred materials of this class include tetra-n-butyl ammonium chloride and the corresponding, especially preferred, bromide. Other phase transfer catalysts are known and suitable examples thereof can be found in, "Applications of Phase-Transfer Catalysts in Organic Synthesis", R. Alan Jones, Aldrichimica Acta, Vol. 9, No. 3, 1976, pp. 35—45.

The amount of phase-transfer catalyst employed can vary with the nature of the particular reactants, the catalyst and desired rate of reaction. Good results can, however, be obtained using from about 2% to about 10% by weight of the combined p-nitrobenzoate salt and dihaloalkane reactants.

The phase-transfer reaction of the invention can be accomplished, for example, by introducing the dihaloalkane reactant into an aqueous medium containing the alkali metal p-nitrobenzoate salt and the phase-transfer catalyst. The reaction is then allowed to occur in the resulting two-phase system. If desired, a two-phase reaction system can be established by contacting the dihaloalkane reactant with an aqueous medium comprising the phase-transfer catalyst and the acid precursor of the p-nitrobenzoate salt reactant. Sufficient alkali (e.g., sodium hydroxide) can then be added with agitation to provide the corresponding salt reactant.

It is to be noted that the process hereof can be accomplished using only water as a solvent material in addition to the reactants and the phase-transfer catalyst. As mentioned previously, the dihaloalkane, the monoester intermediate of formula (IV), and the desired diester product serve to maintain the organic phase during the desired reaction. Accordingly, the use of an extraneous solvent (i.e., a solvent material other than water or the reactants or intermediate products) is not required. Moreover, it will be desired that the process be conducted in the substantial or complete absence of such extraneous solvent. It will be appreciated that the production of the diesters hereof, using only the reactants, the phase-transfer catalyst and water, offers the advantage of simplicity and avoids costs associated with the recovery of organic solvents which are commonly used in organic syntheses.

The amount of water used in the conduct of the process hereof can vary widely. It will be appreciated that sufficient water to dissolve the p-nitrobenzoate or to effect neutralization of the precursor acid should be employed. Similarly, an excessive proportion of water should be avoided so as not to undesirably

decrease the rate of reaction. In general, good results can be achieved using an amount of water corresponding to about 75% to about 200% of the combined weight of the reactants.

In general, the process of the invention will be conducted using substantially equivalent amounts of the p-nitrobenzoate and dihaloalkane reactants. Accordingly, the molar ratio of p-nitrobenzoate salt to dihaloalkane reactant will be about 2:1. A molar proportion substantially less than 2:1 tends to promote formation of impurities such as monoester product while a proportion substantially greater than 2:1 requires the recovery and separation of excess reactant which may otherwise interfere with use of the desired diester product in the production of urethane or other polymeric products. In general, good results can be obtained using a molar proportion of about 2:1, or an excess of either reactant of about 2%.

The phase-transfer catalyzed process of the invention can be operated over a range of temperatures depending upon product requirements, desired rate of reaction, yield, purity or the like. For example, good results are obtained over a range of about 60°C to about 160°C. A preferred temperature range is from about 100°C to about 140°C. If desired, refluxing conditions at a temperature of about 105°C can be conveniently employed. Alternatively, the reaction can be effected at a higher temperature of about 140°C for a greater reaction rate and can be conducted at a pressure of about 40 p.s.i.g.

If desired, water-soluble salts can be added to the aqueous phase to promote the desired phase-transfer catalyzed reaction. Alkali metal bromide salts (e.g., sodium bromide) can be used for this purpose. It has been known in phase-transfer catalyzed reactions to incorporate water-soluble salts into the aqueous phase for purpose of shifting the reaction equilibria in favor of the desired reaction in the organic phase. It has been found in connection with the reaction of the present process that sodium bromide can be added to the aqueous phase to increase the desired rate of reaction. The addition of sodium chloride, by contrast, has been found to decrease the desired rate of reaction. Accordingly, the employment of sodium chloride is, in general, to be avoided.

The practice of the present invention can be appreciated from the following Examples. These Examples are intended merely to be illustrative and not limiting of the present invention.

Example 1

A slurry of 41.78 grams (0.250 mole) of p-nitrobenzoic acid in 100 mls. of water was heated to 50°C with stirring under nitrogen. Twenty grams (0.250 mole) of 50% (by weight) sodium hydroxide solution was added dropwise. The resulting solution (a solution of sodium p-nitrobenzoate of pH 9—10) was heated to 100°C, and allowed to react for 15 minutes. The solution was cooled to 50°C and 4.03 grams (0.0125 mole) tetrabutylammonium bromide and 19.68 grams (0.125 mole) 1-bromo-3-chloropropane were added. The mixture was heated to reflux, 104—105°C, and held there for four hours. After cooling the mixture to 25°C, the product was isolated by filtration, was washed with 500 mls of water, and was dried at 60°C under vacuum to constant weight. The isolated yield was 90—95%.

Example 2

A slurry of 41.78 grams (0.250 mole) of p-nitrobenzoic acid in 100 mls. of water was heated to 50°C with rapid stirring under nitrogen. Twenty grams (0.250 mole) of 50% (by weight) sodium hydroxide was added slowly. The resulting solution (a solution of p-nitrobenzoate of pH 9—10) was heated to 100°C and allowed to react for 15 minutes. The solution was cooled to 50°C and 121 grams (1.17 mole) sodium bromide were added. Tetrabutylammonium bromide (4.03 grams; 0.0125 mole) and 19.68 grams (0.125 mole) of 1-bromo-3-chloropropane were charged. The mixture was heated to reflux, 118°C, and reacted for 2.5 hours, at which point the reaction was complete, as determined by HPLC analysis of the organic phase. The mixture was cooled to 25°C. The product was isolated by filtration, washed with 500 mls. of water, and dried at 60°C under vacuum to constant weight. The isolated yield was about 90%.

Example 3

The experimental procedure described in Example 1 was repeated, except that, 5.05 grams (0.0125 mole) of trioctylmethylammonium chloride (Aliquot 336) was charged in place of the recited tetrabutylammoniumbromide phase-transfer catalyst. Yield was 90—95%. The product contained a residual content of the catalyst.

Example 4

To a 300-ml. Parr Pressure Reactor were charged 16.18 grams (0.202 mole) of 50% (by weight) sodium hydroxide, 33.8 grams (0.202 mole) of p-nitrobenzoic acid, and 76 mls. of water. The pressure reactor was sealed and heated to 100°C with agitation set to 20 rpm for 15 minutes. 3.26 grams (0.010 mole) of tetrabutylammonium bromide were dissolved in 5 mls. of water and charged to the vessel. 15.92 grams (10 mls; 0.101 mole) of 1-bromo-3-chloropropane were charged. The agitation was increased to 50 rpm and the mixture was heated to 130°C (~40 psig). Agitation was decreased to 40 rpm and the reaction mixture was cooled to 25°C. The product was isolated by filtration, washed with 250 mls. of water, and dried at 60°C under vacuum to constant weight. The yield was 90—93%.

Example 5

The experimental procedure described in Example 4 was repeated, except that, an emulsion, comprised of 4.09 grams (0.0101 mole) of trioctylmethylammonium chloride (Aliquot 336) in 5 mls. of water, was charged in place of the tetrabutylammonium bromide solution. The yield was 94—96%.

The p-nitrobenzoate esters prepared by the process of the invention can be used for the production of the corresponding p-aminobenzoate esters which find application as chain-extending or curing agents in the manufacture of polyurethane elastomers. Conversion of the p-nitrobenzoate esters to the p-aminobenzoate esters can be effected by hydrogenation. A suitable method for the conversion and the use of the p-aminobenzoate esters in the production of polyurethane elastomers are described in the aforementioned patent of L. D. Cerankowski et al. (U.S. Patent No. 3,932,360, issued Jannuary 13, 1976).

## Claims

1. A process for preparing a diester of the formula

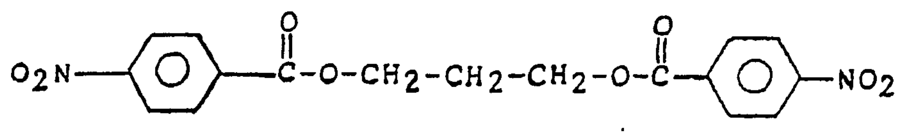

said process comprising reacting, in an aqueous-and-organic two-phase reaction medium, a reactant salt of the formula

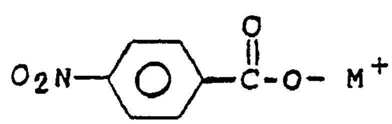

wherein $M^+$ is an alkali metal, and substantially an equivalent amount of a dihaloalkane reactant of the formula

$$X^1—CH_2—CH_2—CH_2—X^2$$

wherein each of $X^1$ and $X^2$ is halo; said aqueous-and-organic two-phase reaction medium being formed without an extraneous organic solvent by contacting water, said reagent salt and said dihaloalkane reagent, and being maintained during reaction by the formation of said diester reaction product; said reaction in said two-phase reaction medium being conducted in the presence of a phase-transfer catalyst.

2. The process according to claim 1 wherein said $M^+$ is sodium.

3. The process according to claims 1 or 2 wherein $X^1$ is chloro and $X^2$ is bromo.

4. The process according to any one of claims 1—3 wherein said water comprises from about 75% to about 200% of the combined weight of said reactants.

5. The process according to any one of claims 1—4 wherein the molar ratio of said reactant salt to said dihaloalkane reactant is about 2:1.

6. The process according to any one of claims 1—5 wherein said phase-transfer catalyst is a quaternary ammonium or phosphonium salt, preferably a tetraalkylammonium halide salt.

7. The process of claim 6 wherein said phase-transfer catalyst is selected from the group consisting of tributylmethylammonium chloride, tetrabutylammonium bromide, trioctylmethylammonium chloride and hexadecyltrimethylammonium chloride.

8. The process according to any one of claims 1—7 wherein said reactants are reacted in said reaction medium at a temperature in the range of about 60°C to about 160°C, preferably of about 100°C to about 140°C.

9. The process according to any one of claims 1—8 wherein an alkali metal bromide salt is present in said aqueous phase.

## Patentansprüche

1. Verfahren zur Herstellung eines Diesters der Formel

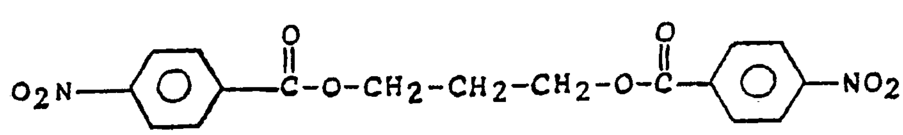

wonach in einem wäßrig-organischen Zweiphasen-Reaktionsmedium ein Reaktanten-Salz der Formel

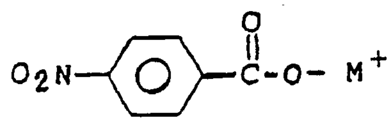

worin $M^+$ ein Alkalimetall darstellt, und im wesentlichen eine äquivalente Menge eines Dihaloalkan-Reaktanten der Formel

$$X^1—CH_2—CH_2—CH_2—X^2$$

umgesetzt wird, worin $X^1$ und $X^2$ jeweils Halogen bedeuten; wobei das wäßrig-organische Zweiphasen-Reaktionsmedium ohne ein äußeres organisches Lösungsmittel gebildet wird, indem Wasser, das Reaktanten-Salz und das Dihalogenalkan-Reagens miteinander in Berührung gebracht werden und während der Reaktion unter Bildung des Diester-Reaktionsproduktes gehalten werden; wobei die Reaktion in dem Zweiphasen-Reaktionsmedium in Gegenwart eines Phasenübertragungskatalysators durchgeführt wird.

2. Verfahren nach Anspruch 1, worin $M^+$ Natrium darstellt.

3. Verfahren nach Anspruch 1 oder 2, worin $X^1$ Chlor und $X^2$ Brom darstellt.

4. Verfahren nach einem der Ansprüche 1 bis 3, worin das Wasser etwa 75% bis etwa 200% des kombinierten Gewichts der Reaktanten ausmacht.

5. Verfahren nach einem der Ansprüche 1 bis 4, worin das Molverhältnis zwischen dem Reaktanten-Salz und dem Dihalogenalkan-Reaktanten etwa 2:1 beträgt.

6. Verfahren nach einem der Ansprüche 1 bis 5, worin der Phasenübertragungs-Katalysator ein quaternäres Ammonium- oder Phosphoniumsalz, vorzugsweise ein Tetraalkylammoniumhalogenid-Salz darstellt.

7. Verfahren nach Anspruch 6, worin der Phasenübertragungskatalysator aus der Gruppe, bestehend aus Tributylmethylammoniumchlorid, Tetrabutylammoniumbromid, Trioctylmethylammoniumchlorid und Hexadecyltrimethylammoniumchlorid ausgewählt ist.

8. Verfahren nach einem der Ansprüche 1 bis 7, worin die Reaktanten in dem Reaktionsmedium bei einer Temperatur im Bereich von etwa 60°C bis etwa 160°C, vorzugsweise von etwa 100°C bis etwa 140°C umgesetzt werden.

9. Verfahren nach einem der Ansprüche 1 bis 8, worin ein Alkalimetallbromidsalz in der wäßrigen Phase vorhanden ist.

**Revendications**

1. Un procédé pour préparer un diester de formule

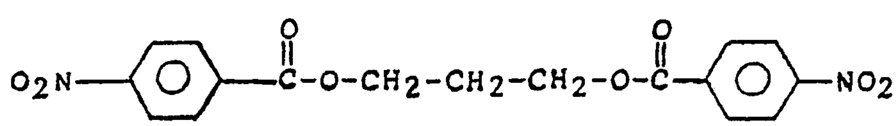

ledit procédé comprenant la mise en réaction, dans un milieu réactionnel diphasique aqueux-organique, d'un sel réactif de formule

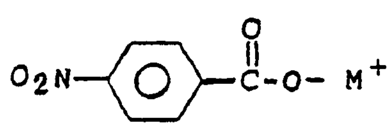

dans laquelle $M^+$ est un métal alcalin, et d'une quantité globalement équivalente d'un réactif dihalogénoalcane de formule

$$X^1—CH_2—CH_2—CH_2—X^2$$

dans laquelle chacun des $X^1$ et $X^2$ est un atome d'halogne; ledit milieu réactionnel diphasique aqueux-organique étant formé, sans solvant organique étranger, par mise en contact de l'eau, dudit sel réactif et dudit réactif dihalogénoalcane, et étant entretenu pendant la réaction par la formation dudit produit réactionnel diester; ladite réaction dans ledit milieu réactionnel diphasique étant effectuée en présence d'un catalyseur par transfert de phase.

2. Le procédé selon la revendication 1, dans lequel ledit $M^+$ est le sodium.

3. Le procédé selon la revendication 1 ou 2, dans lequel $X^1$ est un atome de chlore et $X^2$ est un atome de brome.

4. Le procédé selon l'une quelconque des revendication 1 à 3, dans lequel ladite eau représente entre environ 75% et environ 200% du poids combiné desdits réactifs.

5. Le procédé selon l'une quelconque des revendications 1 à 4, dans lequel le rapport molaire dudit sel réactif audit réactif dihalogénoalcane est d'environ 2:1.

6. Le procédé selon l'une quelconque des revendications 1 à 5, dans lequel ledit catalyseur par transfert de phase est un sel d'ammonium ou de phosphonium quaternaire, de préférence un sel halogénure de tétraalkylammonium.

7. Le procédé de la revendication 6, dans lequel ledit catalyseur par transfert de phase est choisi dans le groupe ainsi composé: chlorure de tributylméthylammonium, bromure de tétrabutylammonium, chlorure de trioctylméthylammonium et chlorure d'hexadécyltriméthylammonium.

8. Le procédé selon l'une quelconque des revendications 1 à 7, dans lequel lesdits réactifs sont mis en réaction dans ledit milieu réactionnel à une température comprise entre environ 60°C et environ 160°C, de préférence entre environ 100°C et environ 140°C.

9. Le procédé selon l'une quelconque des revendications 1 à 8, dans lequel un sel bromure de métal alcalin est présent dans ladite phase aqueuse.